# EUROPEAN PATENT SPECIFICATION

(11) **EP 1 786 391 B1**
(45) Date of publication and mention of the grant of the patent: **12.02.2014**
(21) Application number: 05759587.8
(22) Date of filing: 14.07.2005
(51) Int. Cl.: A61K 8/84, A61Q 17/00

(54) **COMPOSITION FOR COMBATING MSRA ON HANDS COMPRISING POLYHEXAMETHYLENEBIGUANIDE**
ZUSAMMENSETZUNG MIT POLYHEXAMETHYLENBIGUANID ZUR BEKÄMPFUNG VON MSRA AUF HÄNDEN
COMPOSITION DESTINÉE À COMBATTRE LES SARM SUR LES MAINS COMPRENANT DE LA POLYHEXAMETHYLÈNEBIGUANIDE

(30) Priority: 05.08.2004 GB 0417415; 23.11.2004 GB 0425737
(43) Date of publication of application: 23.05.2007
(73) Proprietor: Ebiox Limited, 17 Chesford Grange, Woolston Warrington, WA1 4RQ (GB)
(72) Inventor: MACGREGOR, Keith Martin, Leeds LS1 4HR (GB)
(74) Representative: Smithson, Robert Alan
(86) International application number: PCT/GB2005/002755
(87) International publication number: WO 2006/013315

(56) References cited:
- EP-A- 0 161 425
- FR-A- 2 769 228
- GB-A- 1 544 977
- US-A- 4 420 484
- US-A- 4 587 266
- US-A- 6 045 817
- US-A1- 2002 022 660
- DATABASE EPODOC EUROPEAN PATENT OFFICE, THE HAGUE, NL; XP002346468 & RU 2 207 154 C1 (OBSHCHESTVO S OGRANICHENNOJ OTVETSTVENNOST'JU "MK) 27 June 2003 (2003-06-27)

## Description

The present invention is directed toward a sanitising hand treatment.

The spread of microbial pathogens - particularly bacteria - in many industries is a known problem. For example, in medical facilities, the spread of pathogens can lead to cross contamination between patients thereby promoting the spread of disease. A further example is the food preparation industry where the spread of pathogens can lead to contamination or premature spoilage of food.

In principle the spread of pathogens could be counteracted, by the maintenance of a high standard of hygiene by people who are likely to come in contact with such pathogens. This requires that parts of the body (usually hands) which may have contacted such pathogens are meticulously cleaned. Known ways to achieve such high levels of cleanliness includes the regular washing of the hands with antibacterial soaps, and the regular administration of antibacterial creams, which may have a residual sanitising effect.

Such creams and soaps known in the art achieve their antibacterial efficacy because they contain high levels of alcohol, in particular, isopropyl alcohol. For example, typically, such creams and soaps contain around 60% isopropyl alcohol. However, while isopropyl alcohol has established antibacterial properties, it has the disadvantage that, when used regularly, it can cause skin irritation. As a result personnel may be reluctant to use such creams and soaps.

Thus, in The International Review of Patient Care 2004 '(the official yearbook of the International Hospital Federation, editor Dr Sabine Kühn, published by Frost & Sullivan), it is written:
"Alcohol-based hand rubs (ABHR) are acknowledged as being far superior to other methods of hand disinfection. Yet healthcare facilities still face the challenge of identifying a product that fulfils all their requirements - a product that offers a broad spectrum of activity, high-level efficacy, good skin tolerability and encourages staff willingness to maintain or even improve compliance".

US-B-6,045,817 discloses an antibacterial cleaning composition comprising, in defined amounts, a cationic polymer, a zwitterionic surfactant, a biguanide compound and, as optional components, a nonionic surfactant and a polymeric biocide component. The composition typically comprises water or another non-alcohol solvent. Activity is shown against the bacteria *S*. *aureus, E. hirae, E. coli* and *P*. *aeruginosa.*

US-A-2002/0022660A1 discloses an antimicrobial composition e.g. for use as a surgical scrub. The composition comprises: an alcohol; an effective amount of a cationic quaternary ammonium compound, phenoxy ethanol, and optionally a biguanide compound; and an effective amount of a surfactant system, not including anionic surfactants. Activity is shown against the bacteria *A*. *niger, C*. *albicans, E. faecalis, E. Faecium, E. coli, P. aeruginosa, S. aureus* (#9027), *S. aureus* (MRSA-C1) and *S. epidermidis.*

It is an aim of embodiments of the present invention to address the above mentioned problems and provide a composition which combats pathogens (for example eradicates or disables existing pathogens and/or has prophylactic action against pathogens), yet which has good skincare properties.

According to the present invention there is provided a hand rub or hand wash composition comprising 0.001 - 5 % w/w of polyhexamethylenebiguanide (PHMB) and no C1-C6 mono- or di-alcohol or an amount thereof not greater than 15% w/w, for combating methicillin-resistant *Staphylococcus aureus* on hands.

By hand rub composition herein we mean a composition which is rubbed into the hands, and is not intended to be washed from the hands, but is left on the hands for an extended period, or is absorbed into the skin of the hands.

By hand wash composition herein we mean a composition which is rubbed onto the hands, and is washed from the hands immediately afterwards. An alternative name is hand water-wash composition. In operating theatres a hand wash composition may be called a hand scrub.

When there is more than one biguanide compound present the amounts stated in this specification refer to the total amounts thereof. The same applies to other, optional components described hereafter, for example to quaternary ammonium compound(s), alcohol(s) and to surfactant(s).

Preferably the polyhexamethylenebiguanide (PHMB) is present in an amount in the range 0.01 - 4 % w/w, more preferably in an amount in the range 0.01 - 3 % w/w, more preferably in an amount in the range 0.05 - 2 % w/w, most preferably in an amount in the range 0.05 - 1 % w/w, and especially in an amount in the range 0.1 - 0.5 % w/w.

In certain embodiments at least one quaternary ammonium compound may be present, in an amount from 0.001-5% w/w.

Preferably, a quaternary ammonium compound used in the present invention as an antimicrobial agent has the following general formula: 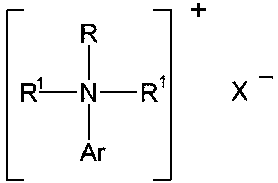 where Ar is an optionally substituted aryl or heteroaryl group, R is any C6 or above unsubstituted branched or linear alkyl group, each group R1 is independently selected from any C1 to C4 branched or unbranched unsubstituted alkyl, and X is a halide anion.

Optional substituents of an aryl or heteroaryl group Ar include halo, cyano, and C1-C4 alkoxy and C1-C4 haloalkyl groups. There may suitably be 1-3 substituents. Preferably, however, Ar is an unsubstituted aryl or heteroaryl group.

Preferably, Ar is selected from optionally substituted phenyl, benzyl, napthyl and pyridyl groups. Most preferably, Ar is an optionally substituted aryl group. Most preferably Ar is an optionally substituted benzyl group.

Preferably, R is any C8 or above unsubstituted branched or linear alkyl group. More preferably, R is any C12 to C20 unsubstituted branched or linear alkyl group. Most preferably, R is any C12 to C20 unsubstituted linear alkyl group. In a particularly preferred embodiment, R is an unbranched unsubstituted C18 alkyl group.

Preferably, R1 are each independently selected from methyl, ethyl, propyl, butyl and isopropyl. More preferably, R1 are each methyl groups.

Preferably, X is a chloride, bromide or iodide anion. Most preferably, X is a chloride anion.

Preferably, the antimicrobial compound comprises benzalkonium chloride (BAC), or may be a derivative thereof.

When a quaternary ammonium compound is present, it is preferably present in an amount in an amount in the range 0.01 - 4 % w/w, more preferably in an amount in the range 0.1 - 3 % w/w, more preferably in an amount in the range 0.5 - 2.5 % w/w, and most preferably in an amount in the range 1 - 2 % w/w.

A composition used in the invention may employ polyhexamethylenebiguanide (PHMB) and no quaternary ammonium compound.

A composition used in the invention may contain polyhexamethylenebiguanide (PHMB) and at least one quaternary ammonium compound.

Preferably, the compositions used in the invention may comprise one or more further components, which we shall call herein auxiliary or carrier material(s), in an amount in the range 80 - 99.989 % w/w.

Preferably, a carrier material may comprise water, but may be any suitable substance for carrying the ingredients to a user, such as an oil etc.

Preferably, there is at least one alcohol present in the composition, preferably in an amount (total amount) in the range 0.01 - 25 % w/w, more preferably in an amount in the range 0.1 - 10 % w/w, and most preferably in an amount in the range 0.5 - 8 % w/w.

Preferably, the at least one alcohol comprises a polyhydric alcohol, preferably having not more than 6 carbon atoms.

Preferably, the at least one alcohol comprises a trihydric alcohol. More preferably, the at least one alcohol comprises glycerol.

When a polyhydric alcohol is present, it is preferably present in an amount in the range 0.1 - 10 % w/w, more preferably in an amount in the range 0.5 - 8 % w/w, more preferably in an amount in the range 1 - 6 % w/w, and most preferably in an amount in the range 1.5 - 5 % w/w; and especially, in an amount in the range 2 - 4 % w/w.

Alternatively or additionally, the at least one alcohol may comprise a viscosity building alcohol.

When a viscosity building alcohol is present, it is preferably present in an amount in the range 0.1 - 10 % w/w, more preferably in an amount in the range 0.5 - 7 % w/w, more preferably in an amount in the range 1 - 5 % w/w, and most preferably in an amount in the range 1.5 - 4 % w/w; and especially, in an amount in the range 2 - 3 % w/w.

Preferably, the viscosity building alcohol comprises a fatty alcohol.

By fatty alcohol it is meant any C8 to C20 branched or unbranched, unsubstituted primary alcohol.

Preferably, the viscosity building alcohol comprises a mixture of fatty alcohols. Preferably, the viscosity building alcohol comprises a cetearyl alcohol.

Alternatively or additionally, the at least one alcohol may comprise an alkoxylated lanolin compound.

When an alkoxylated lanolin compound is present, it is preferably present in an amount in the range 0.05 - 8 % w/w, more preferably in an amount in the range 0.1 - 5 % w/w, more preferably in an amount in the range 0.3 - 3 % w/w, and most preferably in an amount in the range 0.4 - 2 % w/w; and especially, in an amount in the range 0.5 - 1.5 % w/w.

A preferred alkoxylated lanolin compound is an ethoxylated lanolin alcohol.

If a composition used in the invention contains a C1 - C6 mono or di-alcohol, for example a C1-6 alkanol, in particular isopropyl alcohol, it preferably does so in an amount not more than 10 % w/w, more preferably not more than 5 % w/w, more preferably not more than 2 % w/w, and most preferably not more than 0.5 % w/w. When present such an alcohol may be present in an amount thereof of at least 0.01 % w/w. Preferably however such alcohols are not present.

Preferably, at least one lipogel (lipophilic skin-penetrating gel) is present in the composition. Preferably it is present in an amount in the range 0.01 - 15 % w/w, preferably in an amount in the range 0.1 - 12 % w/w, more preferably in an amount in the range 0.5 - 10 % w/w, more preferably in an amount in the range 1 - 8 % w/w, and most preferably in an amount in the range 2 - 6 % w/w; and especially, in an amount in the range 3 - 5 % w/w.

Preferably, the at least one lipogel comprises glyceryl monostearate or cetearyl alcohol.

Preferably, at least one oil is present in the composition. Preferably the composition contains 0.01-15 % w/w of at least one oil. A suitable oil may be a mineral oil. Preferably, the mineral oil comprises paraffin oil.

When a mineral oil is present, it is preferably present in an amount in the range 0.05 - 12 % w/w, more preferably in an amount in the range 0.1 - 10 % w/w, more preferably in an amount in the range 0.5 - 8 % w/w, more preferably in an amount in the range 1.5 - 7 % w/w, and most preferably in an amount in the range 2 - 6 % w/w; and especially, in an amount in the range 4 - 5 % w/w.

Alternatively or additionally, the at least one oil comprises a non-drying oil. Preferably, the non-drying oil comprises castor oil or a derivative thereof, for example an alkoxylated castor oil. More preferably, the non-drying oil comprises PEG40 castor oil.

When a non-drying oil is present, it is preferably present in an amount in the range 0.01 - 10 % w/w, more preferably in an amount in the range 0.05 - 7 % w/w, more preferably in an amount in the range 0.1 - 5 % w/w, more preferably in an amount in the range 0.3 - 3 % w/w, and most preferably in an amount in the range 0.5 - 2 % w/w; and especially, in an amount in the range 0.5 - 1.5 % w/w.

Preferably, the composition further comprises at least one preservative.

When a preservative is present, it is preferably present in an amount in the range 0.001 - 5 % w/w, more preferably in an amount in the range 0.005 - 1 % w/w

A preservative may comprise 1,2-dibromo-2,4-dicyanobutan and/or 2-phenoxyethanol (for example as sold as Euxyl K400®). Such a preservative may preferably be used in an amount in the range 0.01 - 0.5 % w/w; especially in an amount in the range 0.05 - 0.15 % w/w.

A preservative may comprise a paraben and/or 2-phenoxyethanol. By a paraben it is meant any of methyl, ethyl, propyl or butyl esters of para-hydroxybenzoic acid. Preferably, a mixture of paraben and phenoxyethanol is used, as sold under the trade mark Phenonip®. Such a preservative may be present in an amount in the range 0.005 - 5 % w/w, more preferably in an amount in the range 0.01 - 3 % w/w, more preferably in an amount in the range 0.05 - 2 % w/w, and most preferably in an amount in the range 0.1 - 1 % w/w; and especially, in an amount in the range 0.3 - 0.8 % w/w.

Preferably, the composition further comprises fragrance. Preferably, the fragrance is present in an amount in the range 0.05 - 1 % w/w, most preferably in an amount in the range 0.1 - 0.5 % w/w.

Preferably, a composition further comprises at least one colouring agent.

When an at least one colouring agent is present, it is preferably present in an amount in the range 0.001 - 5 % w/w, most preferably in an amount in the range 0.01 - 0.5 % w/w; especially in an amount in the range 0.05 - 0.15 % w/w.

A composition used in the invention may contain at least one detergent agent, preferably in an amount in the range 1 - 400.

Preferably, the at least one detergent agent is present in an amount in the range 10 - 35 % w/w, more preferably in an amount in the range 15 - 32 % w/w, more preferably in an amount in the range 20 - 30 % w/w, and most preferably in an amount in the range 22 - 28 % w/w; and especially, in an amount in the range 24 - 26 % w/w.

Preferably, the at least one detergent agent comprises a metal salt of a C8 - C16 alkyl sulphate. Preferably, the at least one foaming agent comprises an alkali metal salt of a C8 - C16 alkyl sulphate. Preferably, the at least one foaming agent comprises a metal salt of a C10 - C14 alkyl sulphate. More preferably, the at least one foaming agent comprises an alkali metal salt of a C10 - C14 alkyl sulphate. Preferably, the at least one foaming agent comprises a metal salt of a C11 - C13 alkyl sulphate. Preferably, the at least one foaming agent comprises an alkali metal salt of a C11 - C13 alkyl sulphate. Preferably, the at least one foaming agent comprises a metal salt of a C12 alkyl sulphate. Preferably, the at least one foaming agent comprises an alkali metal salt of a C12 alkyl sulphate. Preferably, the alkali metal comprises lithium, sodium or potassium, most preferably, sodium.

Preferably the at least one detergent agent comprises at least one foaming agent.

A particularly preferred detergent agent is sodium lauryl sulphate.

A composition used in the invention may contain at least one amphoteric surfactant, preferably in an amount in the range 0.1 - 15%.

Preferably, the at least one amphoteric surfactant is present in an amount in the range 0.5 - 13 % w/w, more preferably in an amount in the range 1 - 10 % w/w, more preferably in an amount in the range 2 - 8 % w/w, more preferably in an amount in the range 3 - 7 % w/w, and most preferably in an amount in the range 4 - 6 % w/w; and especially, in an amount in the range 4.5 - 5.5 % w/w. Amphoteric surfactants which may be used in the present invention include amphoteric betaine surfactant compounds having the following general formula:

R-N⁺(R₁)₂-R₂COO⁻

wherein R is a hydrophobic group which is an alkyl group containing from 8 to 20 carbon atoms; each R₁ is an alkyl group independently containing from 1 to 3 carbon atoms; and R₂ is an alkylene group containing from 1 to 6 carbon atoms.

Further exemplary useful amphoteric surfactants include those selected from alkylampho(mono)- and (di)-acetates, alkylampho(mono)- and (di)-propionates, and aminopropionates.

Preferably, the at least one amphoteric surfactant comprises a betaine surfactant. Preferably, the at least one amphoteric surfactant comprises Surfac B4 ®.

A composition used in the invention may contain at least one salt, preferably present in an amount in the range 0.01 - 10 % w/w, more preferably in an amount in the range 0.05 - 5 % w/w most preferably in an amount in the range 0.1 - 2 % w/w; especially in an amount in the range 0.5 - 1.5 % w/w.

Preferably, the at least one salt comprises an alkali metal/halide salt. Examples of suitable salts include but are not restricted to sodium chloride, potassium chloride, sodium bromide, potassium bromide, potassium iodide and sodium iodide. A particularly preferred salt is sodium chloride.

Preferably, a hand rub composition used in the invention is a skin-moisturising antimicrobial hand rub composition. Preferably, a hand rub composition used in the invention is a hand-moisturising composition preferably formulated as a gel, cream or lotion.

The present invention combats methicillin-resistant *Staphylococcus aureus* (MRSA). Preferably it also shows efficacy in the treatment of *Legionella* (responsible for Legionnaires disease) and *Escherichia coli* (*E*. *coli*). Preferably it is also effective against one or both of *Pseudomonas aeruginosa* and *Enterococcus hirae.* The combating of these further bacteria represent preferred aspects of the present invention.

All of the features described herein may be combined with any of the above aspects, in any combination.

The present invention will now be illustrated in the following examples.

### Test compositions

### Composition 1 (hand wash):

| | |
|---|---|
| Purified Water | 68.3%w/w |
| 5 p/w Vantocil® (20% aqueous solution of PHMB) | 0.5%w/w |
| Surfac B4 (surfactant, viscosity modifier) | 5.0%w/w |
| Empilan® CDE (cocamide - non-polymeric thickener) | 2.0%w/w |
| Glycerol | 2.0%w/w |
| Sodium chloride | 1.0%w/w |
| Fragrance | 0.3%w/w |
| Keltrol® RD xanthan gum (polysaccharide thickener) | 0.5%w/w |
| Euxyl® K400 (preservative) | 0.1%w/w |
| FD & C red colour | 0.3%w/w |

### Composition 2 (hand rub):

| | |
|---|---|
| Purified Water | 86.8%w/w |
| Vantocil® (20% aqueous solution of PHMB) | 1.0%w/w |
| Alkyldimethylbenzylammonium chloride | 1.0%w/w |
| Didecyldimethylbenzylammonium chloride | 0.5%w/w |
| Mineral oil (light grade) | 1.0%w/w |
| PEG 100 stearate (LEXEMUL® 561) | 4.0%w/w |
| Polyoxyethylene (21) stearyl ether (BRIJ® 721) | 2.0%w/w |
| Polydimethylsiloxane (Dow Corning 200 fluid) | 1.0%w/w |
| Glycerol | 2.0%w/w |
| Phenonip (paraben preservative) | 0.6%w/w |
| Imidazolidinyl urea | 0.1%w/w |

### Examples Set 1

### Test Method

PrEn 12054 Quantitative suspension test for the evaluation of bactericidal activity of products for hygienic and surgical hand rub and hand wash used in human medicine (phase 2/step 1).

### Test organisms

| | |
|---|---|
| *Staphylococcus aureus* | NTC 10788 |
| *Pseudomonas aeruginosa* | NTC 6749 |
| *Escherichia coli* | NTC 10418 |
| *Enterococcus hirae* | NTC 12367 |
| *MRSA* | NTC 12493 |

### Test requirements - Hygienic Hand rub

To pass this test the product is required to demonstrate a 10⁵ reduction in viable microbe count after 1 minute, and also after 30 seconds. The product is tested undiluted.

### Test requirements - Hygienic Hand wash

To pass this test the product is required to demonstrate a 10³ reduction in viable microbe count after 1 minute, and also after 30 seconds. A 55% dilution of the product was tested.

| | |
|---|---|
| **Contact time** | 30 seconds, 1 minute |
| **Test temperature** | 20°C |
| **Inhibition method** | Dilution/neutralization |
| | |
| **Neutraliser** | Tween® 80 40g/l, sodium lauryl sulphate 10g/l, Lecithin 4g/l, sodium thiosulphate 5g/l, Saponin 30 g/l. |

Tests were performed to establish the suitability of this neutralizer in neutralizing the activity of the disinfectant without being inhibitory to the test organisms. Initial tests were carried out with the standard neutralizer described in EN 1276 but this proved unsatisfactory as a neutralizer. An increase in the Tween 80 and Lecithin concentration and the addition of Sodium Lauryl Sulphate was required.

### Summary of Test Method

The test method involves mixing 1ml of the test bacteria with 9ml of disinfectant. After the required contact time, 1ml is removed to 9ml of recovery/neutralizer, which is then diluted/plated to detect surviving test bacteria.

### Results

### Bactericidal Activity of Composition 1 using suspension test prEN 12054

### Log10 reductions achieved in 30 seconds and 1 minute. (Tests carried out in duplicate)

| Test organism | Log₁₀ initial count (challenge) | Contact time | |
|---|---|---|---|
| | | 30 seconds | 1 minute |
| *Ps. aeruginosa* | 6.92 | 4.86 | 5.74 |
| *Esch. coli* | 6.92 | >5.92 | >5.92 |
| *Staph. aureus* | 6.81 | >5.81 | >5.81 |
| *Ent. hirae* | 7.08 | >6.08 | >6.08 |
| *MRSA* | 6.95 | >6.95 | >6.95 |

### Bactericidal Activity of Composition 2 using suspension test prEN 12054

### Log10 reductions achieved in 30 seconds and 1 minute. (Tests carried out in duplicate)

| Test organisms | Log₁₀ initial count (challenge) | Contact time | |
|---|---|---|---|
| | | 30 seconds | 1 minute |
| *Ps. aeruginosa* | 6.23 | >5.23 | >5.23 |
| *Esch. coli* | 6.95 | >5.95 | >5.95 |
| *Staph. aureus* | 7.04 | >6.04 | >6.04 |
| *Ent. hirae* | 6.41 | >5.41 | >5.41 |
| *MRSA* | 7.11 | >6.11 | >6.11 |

### Conclusion

When tested in accordance with prEN 12054, Composition 1 and Composition 2 possess bactericidal activity at 20C. A >log10 (99.999%) reduction was achieved with all test organisms i.e. *Ps. aeruginosa, Esch. coli, Staph. aureus, Ent. hirae, MRSA* in 1 minute. To satisfy the requirements for the test, at least a 5 log10 reduction in specified test organisms is required within 1 minute for the hygienic hand rub formulation and at least a 3 log10 reduction in 1 minute for the hygienic hand wash formulation. Both compositions also passed the test at 30 seconds.

### Examples set 2 - Hand wash composition

Further testing was carried out in accordance with standard test EN 1499 (Phase 2, step 2) as follows.

The test organism was *Escherichia coli* K12 NCTC 10538.

EN 1499 (Phase 2 step 2) - chemical disinfectants and antiseptics - test for the evaluation of bactericidal activity of skin disinfectants, simulating practical conditions for establishing whether a product is suitable for hygienic hand wash were disinfection is medically indicated, or in food, industrial, domestic and institutional areas.

The test comprises of an assessment of the number of test organisms (*E. coli*) released from the fingertips of artificially contaminated hands of volunteers, before and after hygienic hand washing with test reference products. The ratio of the two resulting values is called the reduction factor (RF). It represents a measure of the antimicrobial efficacy of the hand wash products tested. To pass the test, the RF of the test products should be significantly superior to the reference product i.e. European standard soft soap.

### Reference hand wash procedure

5ml of the reference product i.e. European standard soft soap is poured into the pre-moistened cupped hands, and rubbed vigorously into the skin for 30 seconds up to the wrists in accordance with a standard hand wash procedure to ensure total coverage of the hands. The standard procedure comprises five strokes backwards and forwards, palm to palm, right palm over left dorsum and left palm over right dorsum, palm to palm with fingers interlaced, back of fingers to opposing palms with fingers interlocked, rotational rubbing of right thumb clasped in left palm and left thumb clasped in right palm,' rotational rubbing with clasped fingers of right hand in palm of left hand and clasped fingers of left hand in palm of right hand. The procedure is then repeated to give a total rubbing time of 60 seconds.

The reference procedure is completed by a 15 second water rinse of the fingers from distal to proximal with fingertips upright, under running tap water. The hands are held with the fingers pointing upwards until excess water is dried off by the experimenter, using two dry paper towels to dab off any excess water from the base .of the hands and the wrists. The hands are then sampled immediately by rubbing the fingertips and thumb for one minute on the base of a Petri dish containing 10ml of TSB (tryptic soy broth).

### Test hand wash procedure

2ml of the test product (i.e. hand wash Composition 1 described above) is applied to the hands and rubbed as described above for the reference product. The hands are washed and tested, also as described above for the reference product.

### Neutraliser

Quantities specified are per litre and are made up in TSB.

| | |
|---|---|
| 4g | Lecithin |
| 40g | Tween 80 (Polysorbate 80) |
| 5g | Sodium thiosulphate |
| 1g | L-Histidine |
| 30g | Saponin |
| 10g | Lauryl sulphate |

This was established as an effective neutraliser prior to commencement of the test.

### Results

The number of colony forming units of the test bacteria released from the fingertips of left and right hands of 14 volunteers before and after applying the reference product and Composition 1 were determined.

The results were statistically analysed using the Wilcoxon matched-pairs signed-ranks test. Composition 1 applied as a 2ml aliquot was determined to be significantly more effective (p = 0.01) than the reference product i.e. European standard soft soap applied in one 5ml volume, both being applied over 60 seconds and rinsed off for 15 seconds.

The external test house which conducted these tests concluded:
"Using the methodology described in the European Standard EN 1499 (1997) "Ebiox hygienic hand wash^{†}" ref. EBX HS6, applied in accordance with the manufacturer's instructions i.e. as a single application consisting of 2ml of product over a 60s period, is significantly more effective than the reference product, i.e. European standard soft soap applied as a single 5ml aliquot over 60s. Both products i.e. test and reference were followed by a 15s rinse under running tap water followed by drying the wrists, as defined in EN 1499.
To conform to the European efficacy standard EN 1499, the products under test must be significantly superior to the reference product. "Ebiox hygienic hand wash^{†}" (Ref EBX HS6) when applied as a single 2ml application over 60s as instructed by the manufacturer, therefore, DOES conform to European Efficacy Standard EN 1499".
^{†} that is, Composition 1 identified above

### Example set 3 - Hand rub

Further testing was carried out on the hand rub, Composition 2, described above.

The test organism was *Escherichia coli* K12 NCTC 10538

EN 1500 (Phase 2 step 2) chemical disinfectants and antiseptics - test for the evaluation of bactericidal activity of skin disinfectants, simulating practical conditions for establishing whether a product is suitable for hygienic hand rub where disinfection is medically indicated, or in food, industrial, domestic and institutional areas.

The test comprises of an assessment of the number of test organisms (*E. coli*) released from the fingertips or artificially contaminated hands of volunteers, before and after hygienic hand rub with test and reference products. The ratio of the two resulting values is called the reduction factor (RF). It represents a measure of the antimicrobial efficacy of the hand rub products tested. To pass the test, the RF of the test products should not be significantly inferior to the reference product i.e. 60% propan-2-ol. Each of 15 volunteers is made to assess the product and the reference product using the same batch of contamination fluid, although a different batch may be used for each volunteer. Approximately half the volunteers assess the test product first, followed by the reference product while the remaining volunteers assess the reference product first.

Prior to contamination, the hands are washed for one minute using European Standard Soft Soap. After thoroughly drying, the fingers are then contaminated by immersion of the hands up to the mid metacarpals into a bowl containing 2 litres of contamination fluid, i.e. an overnight culture of *E*. *coli* K12 NCTC 10538 in TSB. After 5 seconds, the hands are withdrawn from the contamination fluid, excess fluid is allowed to drip from the fingers, and then the hands are held horizontally with the fingers spread apart and allowed by dry for 3 minutes. The fingertips are then sampled to obtain "Pre-valued" of surviving test organisms before applying the "Test" or "Reference" procedure.

### European Standard Reference Hand Rub Procedure

3ml of propan-2-ol (60% v/v) were poured into the cupped hands, and rubbed vigorously into the skin in the same manner and for the same time as is described above for the hand wash test, with the sole exception that after the first 30 seconds of rubbing, a further 3ml of propan-2-ol (60% v/v) was poured into the cupped hands, followed by the next 30 seconds of rubbing (to reach a total rubbing time of 60 seconds).

The reference procedure is completed by a 5 second water rinse of the fingers from distal to proximal with fingertips upright, under running tap water. Excess water is shaken off. The hands are then sampled immediately by rubbing the fingertips and thumb for one minute on the base of two Petri dishes, each containing 10ml of TSB. Each hand is sampled independently in separate Petri dishes.

### Test hand rub procedure

The test product, Composition 2, was applied in the same way as the reference described above (i.e. one 3ml volume rubbed into the hands over a 30 second period followed by a further 3ml application rubbed into the hands over a second 30 second period; 60 seconds rubbing in total).

Rising and sampling then take place as described above for the reference.

### Neutraliser

Quantities specified are per litre and are made up in TSB.

| | |
|---|---|
| 40g | Tween 80 (Polysorbate 80) |
| 4g | Lecithin |
| 5g | Sodium thiosulphate |
| 7g | Sodium lauryl sulphate |
| 30g | Saponin |
| 1g | Histidine |
| 1g | Cysteine |

This was established as an effective neutraliser prior to commencement of the test.

### Results

The number of colony forming units of the test bacteria released from the fingertips of left and right hands of 14 volunteers before and after applying the reference product 60% propan-2-ol and Composition 2 were determined.

The results were statistically analysed using the Wilcoxon matched-pairs signed-ranks test. Composition 2 applied over 30 + 30 seconds as 3ml + 3ml aliquots was determined to be statistically as effective (p = 0.1) as the reference product 60% propan-2-ol, also applied over (30 + 30) seconds as (3 + 3) ml aliquots.

The external test house which conducted these tests concluded:
"Using the methodology described in the European Standard EN 1500 (1997) "Ebiox Esense hand disinfectant^{†}" applied in accordance with the manufacturer's instructions i.e. as a 3ml application rubbed in over 30s followed by a further 3ml application rubbed in over 30s (total rubbing time 60s) when analysed statistically is significantly no less effective than the reference product, i.e. 60% propan-2-ol applied in two x 3ml aliquots over 60s. Both products were followed by a 5s rinse under running tap water as defined in EN 1500.
To conform to the European efficacy standard EN 1500, the products under test must not be significantly inferior to the reference product.
"Ebiox Esense hand disinfectant^{†}" when applied as a 3ml application rubbed in over 30 seconds followed by a further 3ml application rubbed in over 30s (total rubbing time 60s) as instructed by the manufacturer, therefore, conforms to European Efficacy Standard EN 1500".
^{†} that is, Composition 2 identified above..

### Example set 4 - hand rub

Corresponding EN 1500 testing was carried out on the following hand rub compositions:

Composition 3 - same as Composition 2 described above but with the biguanide compound omitted.

Composition 4 - same as Composition 2 described above but with both quaternary ammonium compounds omitted.

The results can be summarised thus. Compositions 3 and 4 both gave a high and statistically supported level of antimicrobial efficacy, but, in neither case was this high enough to reach the standard required to pass the EN 1500 test.

As noted above Composition 2 which contained the biguanide compound and the quaternary ammonium compounds did pass the EN 1500 test.

The EN 1500 test is regarded as a stringent test; as noted above propan-2-ol-based hand rubs are extremely effective in achieving germ kill. The problem with them is not lack of efficacy but lack of comfort in their use. If they are extremely effective but are not used because they cause the skin to become dry or chapped, then in such real-life situations they are of no efficacy at all. Nevertheless EN 1500 looks only to their efficacy and the finding of an hand rub composition which passes the EN 1500 test and which offers the prospect of being much more "skin-kind" - not being based on propan-2-ol - is of potential value.

Thus, in summary compositions in accordance with the present invention have been shown to have excellent bactericidal activity without the disadvantages associated with high alcohol content.

## Claims

1. A hand rub or hand wash composition for use in combating methicillin-resistant *Staphylococcus aureus* on hands comprising 0.001 - 5 % w/w of at least one biguanide compound, and no C1-C6 mono- or di-alcohol or an amount thereof not greater than 15% w/w, wherein the biguanide comprises polyhexamethylenebiguanide (PHMB).

2. A composition as claimed in claim 1 comprising 0.01 - 3% w/w of polyhexamethylenebiguanide (PHMB).

3. A composition as claimed in claim 1 or 2, comprising 0.001 - 5% w/w of at least one quaternary ammonium compound.

4. A composition as claimed in any preceding claim, comprising no C1 - C6 mono- or di-alcohol or an amount thereof not greater than 5% w/w.

5. A composition as claimed in claim 4, comprising no C1 - C6 mono- or di-alcohol.

6. A composition as claimed in any preceding claim, comprising 0.01 - 25 % w/w of at least one alcohol.

7. A composition as claimed in any preceding claim, wherein the alcohol comprises a trihydric alcohol having not more than 6 carbon atoms.

8. A composition as claimed in any preceding claim to additionally combat *Legionella.*

9. A composition as claimed in any preceding claim to additionally combat *Escherichia coli.*

10. A composition as claimed in any preceding claim to additionally combat *Pseudomonas aeruginosa.*

11. A composition as claimed in any preceding claim to additionally combat *Enterococcus hirae.*

## Patentansprüche

1. Handeinreibe- oder Handwaschzusammensetzung zur Verwendung in der Bekämpfung von methicillinresistentem *Staphylococcus aureus* an den Händen, die 0,001 - 5% w/w an mindestens einer Biguanidverbindung und keinen C1-C6-Mono- oder- Dialkohol bzw. eine C1-C6-Mono- oder-Dialkoholmenge, die 15% w/w nicht überschreitet, umfasst, wobei das Biguanid Polyhexamethylenbiguanid (PHMB) umfasst.

2. Zusammensetzung nach Anspruch 1, die 0,01 - 3% w/w Polyhexamethylenbiguanid (PHMB) umfasst.

3. Zusammensetzung nach Anspruch 1 oder 2, die 0,001 - 5% w/w an mindestens einer quartären Ammoniumverbindung umfasst.

4. Zusammensetzung nach einem vorhergehenden Anspruch, die keinen C1-C6-Mono- oder- Dialkohol bzw. eine C1-C6-Mono- oder- Dialkoholmenge, die 5% w/w nicht überschreitet, umfasst.

5. Zusammensetzung nach Anspruch 4, die keinen C1-C6-Mono- oder- Dialkohol umfasst.

6. Zusammensetzung nach einem vorhergehenden Anspruch, die 0,01 - 25% w/w an mindestens einem Alkohol umfasst.

7. Zusammensetzung nach einem vorhergehenden Anspruch, wobei der Alkohol einen dreiwertigen Alkohol mit nicht mehr als 6 Kohlenstoffatomen umfasst.

8. Zusammensetzung nach einem vorhergehenden Anspruch, für die zusätzliche Bekämpfung von *Legionella.*

9. Zusammensetzung nach einem vorhergehenden Anspruch, für die zusätzliche Bekämpfung von *Escherichia coli.*

10. Zusammensetzung nach einem vorhergehenden Anspruch, für die zusätzliche Bekämpfung von *Pseudomonas aeruginosa.*

11. Zusammensetzung nach einem vorhergehenden Anspruch, für die zusätzliche Bekämpfung von *Enterococcus hirae.*

## Revendications

1. Composition de friction des mains ou de lavage des mains pour une utilisation dans la lutte contre *Staphylococcus aureus* résistant à la méthicilline sur les mains comprenant de 0,001 à 5 % p/p d'au moins un composé de biguanide, et ne comprenant pas de C1-C6 mono ou dialcool ou comprenant une quantité de ceux-ci qui n'est pas supérieure à 15 % p/p, le biguanide comprenant du biguanide de polyhexaméthylène (PHMB).

2. Composition selon la revendication 1 comprenant de 0,01 à 3 % p/p de biguanide de polyhexaméthylène (PHMB).

3. Composition selon la revendication 1 ou la revendication 2, comprenant de 0,001 à 5 % p/p d'au moins un composé ammonium quaternaire.

4. Composition selon l'une quelconque des revendications précédentes, ne comprenant pas de C1-C6 mono ou dialcool ou comprenant une quantité de ceux-ci qui n'est pas supérieure à 5 % p/p.

5. Composition selon la revendication 4 ne comprenant pas de C1-C6 mono ou dialcool.

6. Composition selon l'une quelconque des revendications précédentes, comprenant de 0,01 à 25 % p/p d'au moins un alcool.

7. Composition selon l'une quelconque des revendications précédentes, dans laquelle l'alcool comprend un alcool trihydrique ne possédant pas plus de 6 atomes de carbone.

8. Composition selon l'une quelconque des revendications précédentes afin de lutter en outre contre *Legionella*.

9. Composition selon l'une quelconque des revendications précédentes afin de lutter en outre contre *Escherichia coli.*

10. Composition selon l'une quelconque des revendications précédentes afin de lutter en outre contre *Pseudomonas aeruginosa.*

11. Composition selon l'une quelconque des revendications précédentes afin de lutter en outre contre *Enterococcus hirae.*
